# EUROPÄISCHE PATENTSCHRIFT

(11) **EP 0 999 999 B1**
(45) Veröffentlichungstag und Bekanntmachung des Hinweises auf die Patenterteilung: **28.05.2003**
(21) Anmeldenummer: 98938690.9
(22) Anmeldetag: 20.07.1998
(51) Int. Cl.: C07B 39/00, C07C 17/14, C07C 201/12, C07C 67/307, C07C 253/30

(54) **VERFAHREN ZUR HERSTELLUNG VON SUBSTITUIERTEN BENZYLBROMIDEN**
METHOD FOR PREPARING SUBSTITUTED BENZYL BROMIDES
PROCEDE POUR LA PREPARATION DE BROMURES DE BENZYLE SUBSTITUES

(30) Priorität: 30.07.1997 DE 19732693
(43) Veröffentlichungstag der Anmeldung: 17.05.2000
(73) Patentinhaber: BASF AKTIENGESELLSCHAFT, 67056 Ludwigshafen (DE)
(72) Erfinder: WINGERT, Horst, D-68159 Mannheim (DE); GÖTZ, Norbert, D-67547 Worms (DE); KEIL, Michael, D-67251 Freinsheim (DE); MÜLLER, Bernd, D-67227 Frankenthal (DE)
(74) Vertreter: Münch, Volker, Dr.
(86) Internationale Anmeldenummer: EP9804485
(87) Internationale Veröffentlichungsnummer: WO99006339

(56) Entgegenhaltungen:
- EP-A- 0 336 567
- EP-A- 0 553 879
- CH-A- 633 765
- GB-A- 707 990
- PATENT ABSTRACTS OF JAPAN vol. 014, no. 531 (C-0780), 21. November 1990 & JP 02 221233 A (SANKO KAGAKU KOGYO KK), 4. September 1990

## Beschreibung

Die vorliegende Erfindung betrifft ein Verfahren zur Herstellung von substituierten Benzylbromiden der allgemeinen Formel I 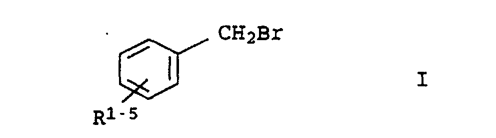 in der mindestens ein Substituent R¹⁻⁵ eine elektronenziehende Gruppe wie Fluor, Chlor, Brom, C₁-C₄-Alkoxycarbonyl, Cyano oder Nitro bedeutet und die übrigen Substituenten R¹⁻⁵ Wasserstoff oder Methyl bedeuten,
durch Bromierung von substituierten Toluolen der allgemeinen Formel II 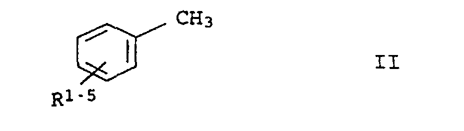 mit einem Bromierungsmittel bei Temperaturen von 20 bis 95°C.

Die Seitenkettenbromierung von Alkylaromaten ist seit langer Zeit bekannt (Houben-Weyl, Band ⁵/₄, S. 331 ff (1960))
In diesem Überblick wird darauf hingewiesen, daß elektronenziehende Substituenten, wie Halogenatome oder Nitrogruppen diese Reaktion erschweren. Sehr schwer substituierbare Verbindungen, speziell Nitrotoluole, lassen sich oft erst bei Temperaturen oberhalb von 100°C und unter Druck zur Reaktion bringen, was auf Grund der geringen thermischen Beständigkeit der Verbindungen erhebliche Sicherheitsprobleme mit sich bringt.

In neuerer Literatur (EP-A 336 567) wird die besonders schwierige Herstellung von o-Nitrobenzylbromid durch Bromierung von o-Nitrotoluol mit Bromwasserstoff in Gegenwart von Wasserstoffperoxid unter Bestrahlung mit Licht beschrieben, wobei Selektivitäten von über 90 % erhalten werden konnten. Nachteilig an diesem Verfahren sind folgende Befunde:

Die Erzeugung der erforderlichen Bromradikale wird durch Bestrahlung mit Licht vorgenommen, was im Dauerbetrieb zur Belegung der Lampen und damit zu erheblichen Beeinträchtigungen führen kann.

Zur Erreichung des Optimums ist man auf den engen Temperaturbereich von 60 - 70°C angewiesen. Gute Selektivitäten werden nur bei relativ geringen Umsätzen erhalten.

Für optimale Reaktionsbedingungen ist das Molverhältnis von Wasserstoffperoxid/Substrat und das Molverhältnis von Wasserstoffperoxid/Bromwasserstoff nur in relativ engen Bereichen variierbar.

Ein weiteres Bromierungsverfahren für an mit elektronenziehenden Gruppen substituierte aromatische Kerne gebundene Methylgruppen wird auch in der Patentschrift JP-A-2 221 233 beschrieben. Die Umsetzung erfolgt mit einem Bromierungsmittel in Gegenwart von wäßrigem H₂O₂ ohne organische Initiatoren.

In der Patentschrift CH-A-633 765 wird die Herstellung von Benzal- und Benzylbromiden mit in o-Stellung elektronenanziehenden Substituenten aus dem entsprechenden, substituierten Toluol beschrieben. Das Verfahren wird in nichtoxidativen Medien mit symmetrisch halogenierten Kohlenwasserstoffen als Lösungsmittel durch Umsetzung mit einer positiven Bromverbindung, wie zum Beispiel N-Bromsuccinimid, in Gegenwart von AIBN als Radikalbildner durchgeführt.

Ein ähnliches Halogenierungsverfahren offenbaren die Patentschrift GB-A-707 990 und EP-A-0 553 879, bei dem die zu halogenierend Verbindung durch Umsetzung mit N-Halogencarbonsäureamiden- oder imiden in Gegenwart von Azoverbindungen in nicht-oxidativen Medien halogeniert wird.

Aus diesen Schriften ist nicht erkennbar, daß die eingestzten Initiatoren ihre Wirkung auch in Gegenwart oxidativer Medien ihre Wirkung behalten.

Es wurde nun gefunden, daß man durch elektronenziehende Gruppen substituierte Benzylbromide mit sehr guten Selektivitäten erhält, wenn die Bromierung in Gegenwart eines Azocarbonsäurenitrils oder eines Azocarbonsäureesters und in Gegenwart eines Oxidationsmittels durchgeführt wird.

Überraschend ist, daß die Generierung von Bromradikalen mittels organischer Initiatoren auch in Gegenwart starker Oxidationsmittel gelingt. Azocarbonsäureester und Azocarbonsäurenitrile. zeichnen sich durch eine besonders hohe Stabilität gegenüber den genannten Oxidationsmitteln aus und sind daher für den Einsatz als Initiatoren im erf indungsgemäßen Prozeß prädestiniert. Das erfindungsgemäße Verfahren bringt eine Reihe von verfahrenstechnischen und ökonomischen Vorteilen, die im folgenden kurz aufgelistet und im weiteren näher erläutert werden:
1. Wegfall der apparativ aufwendigen Bestrahlungstechnik
2. Breite Variationsmöglichkeit des verwendeten Bromierungs- sowie des Oxidationsmittels
3. Vergrößertes Temperaturfenster hin zu niedrigeren Reaktionstemperaturen
4. Die Reihenfolge der Zugabe der einzelnen Reaktionspartner kann umgekehrt werden. Vorlage des zu bromierenden Substrats und des Oxidationsmittels und Zudosierung des Bromierungsmittels. Die Konzentration an korrosivem Bromierungsmittel, vorallem Bromwasserstoff kann auf diese Weise im Reaktionsgefäß sehr klein gehalten werden.

Geeignete Lösungsmittel für das erfindungsgemäße Verfahren sind solche, die sich bei der Bromierung als inert erweisen, wie beispielsweise aromatische Kohlenwasserstoffe wie Benzol, tert.-Butylbenzol und tert.-Amylbenzol, halogenierte Kohlenwasserstoffe wie Methylenchlorid, Chloroform und Chlorbenzol, 1,2-Dichlorethan, Tetrachlorkohlenstoff, Dichlorbenzol oder Trichlorbenzol.

Es können auch Gemische der genannten Lösungsmittel verwendet werden.

Bevorzugt sind halogenierte Kohlenwasserstoffe wie Methylenchlorid, 1,2-Dichlorethan, Chloroform, Tetrachlorkohlenstoff, ortho- oder para-Dichlorbenzol, 1,2,4-Trichlorbenzol und insbesondere Chlorbenzol.

Die im erfindungsgemäßen Verfahren eingesetzten ortho-Nitrotoluole II sind zumeist käuflich erwerblich oder nach in der Literatur beschriebenen Verfahren einfach zugänglich (z.B. Organikum, Barth Verlagsgesellschaft (1993) 320 ff).

Im Gegensatz zur Lehre aus EP-A 336 567 ist man beim Arbeiten nach dem erfindungsgemäßen Verfahren in seiner Ausführung sehr flexibel. Es lassen sich Bromierungsmittel wie elementares Brom, oder Bromsalze wie beispielsweise Natriumbromid u.a. sowie Bromwasserstoff, bevorzugt in Form seiner wäßrigen Lösung, der Bromwasserstoffsäure einsetzen. Besonders bevorzugt werden technische, azeotrope Mischungen der Bromwasserstoffsäure.

Für die Oxidation des Bromwasserstoffs, bzw. der Bromid-Ionen sind beispielsweise Oxidationsmittel wie Persäuren, Peroxide, Hypochlorit (Chlorbleichlauge), Chlor, Natriumbromat und Kaliumperoxodisulfat geeignet, besonders geeignet ist Wasserstoffperoxid.

In einer bevorzugten Ausführungsform des erfindungsgemäßen Verfahrens wird mit solchen Mengen an Oxidationsmittel gearbeitet, daß auch der bei der Reaktion gebildete Bromwasserstoff wieder oxidiert wird. Bevorzugt werden pro Bromidäquivalent 1,5 bis 2,0 Äquivalente (Val) des Oxidationsmittels zugesetzt. Verwendet man hingegen elementares Brom als Bromquelle, so genügt es, 0,5 bis 1,0 Äquivalente (bezogen auf Brom) eines Oxidationsmittels zusetzen. Auf diese Weise läßt sich die Einsatzmenge an Bromierungsmittel nahezu halbieren.

Das Bromierungsmittel wird im allgemeinen in einem Molverhältnis von 0,7 bis 1,3 und bevorzugt in einem Molverhältnis von 0,9 bis 1,0 zum eingesetzten o-Nitrotoluol II eingesetzt.

Ein besonderer Vorteil des erfindungsgemäßen Verfahrens besteht darin, daß als Initiatoren Azoverbindungen wie Azocarbonsäureester und Azocarbonsäurenitrile verwendet werden und damit auf die Einstrahlung von Licht verzichtet werden kann. Diese Initiatoren können problemlos im Edukt oder im Lösungsmittel gelöst werden und dementsprechend vorgelegt oder zudosiert werden. Besonders bevorzugte Azoverbindung ist Azoisobutyronitril (AIBN).

Die Initiatoren werden im allgemeinen in einer Konzentration von 0,1 bis 20 mol.-% bezogen auf die Brom- bzw. Bromidionen-Konzentration (je nach Einsatzstoff) und bevorzugt in einer Konzentration von 1 bis 10 mol.-% dem Reaktionsgemisch zugesetzt.

Die Bromierung wird bei Temperaturen von 20 bis 95°C, bevorzugt bei 20 bis 80°C durchgeführt. Die optimale Reaktionstemperatur richtet sich zum einen nach der thermischen Stabilität des eingesetzten o-Nitrotoluols II und des daraus gewonnenen Produkts III und zum anderen nach der Zerfallstemperatur des Initiators. Die folgende Tabelle gibt eine Übersicht verschiedener Initiatoren, der jeweiligen Struktur und dazugehöriger 10-h-Halbwertszeit-Zerfalltemperatur. Bevorzugt wird bei einer Reaktionstemperatur knapp über oder unterhalb der 10-h-Halbwertszeit-Zerfalltemperatur des Initiators gearbeitet (±10°C). Das Arbeiten unterhalb der 10 h-Halbwertszeit-Zerfallstemperatur führt im allgemeinen zu einem sparsamen Initiatorverbrauch und zu einer höheren Selektivität. Allerdings müssen höhere Reaktionszeiten in Kauf genommen werden. Durch die Wahl eines geeigneten Initiators läßt sich somit die Reaktionstemperatur in weiten Bereichen variieren und den jeweiligen Bedingungen optimal anpassen.

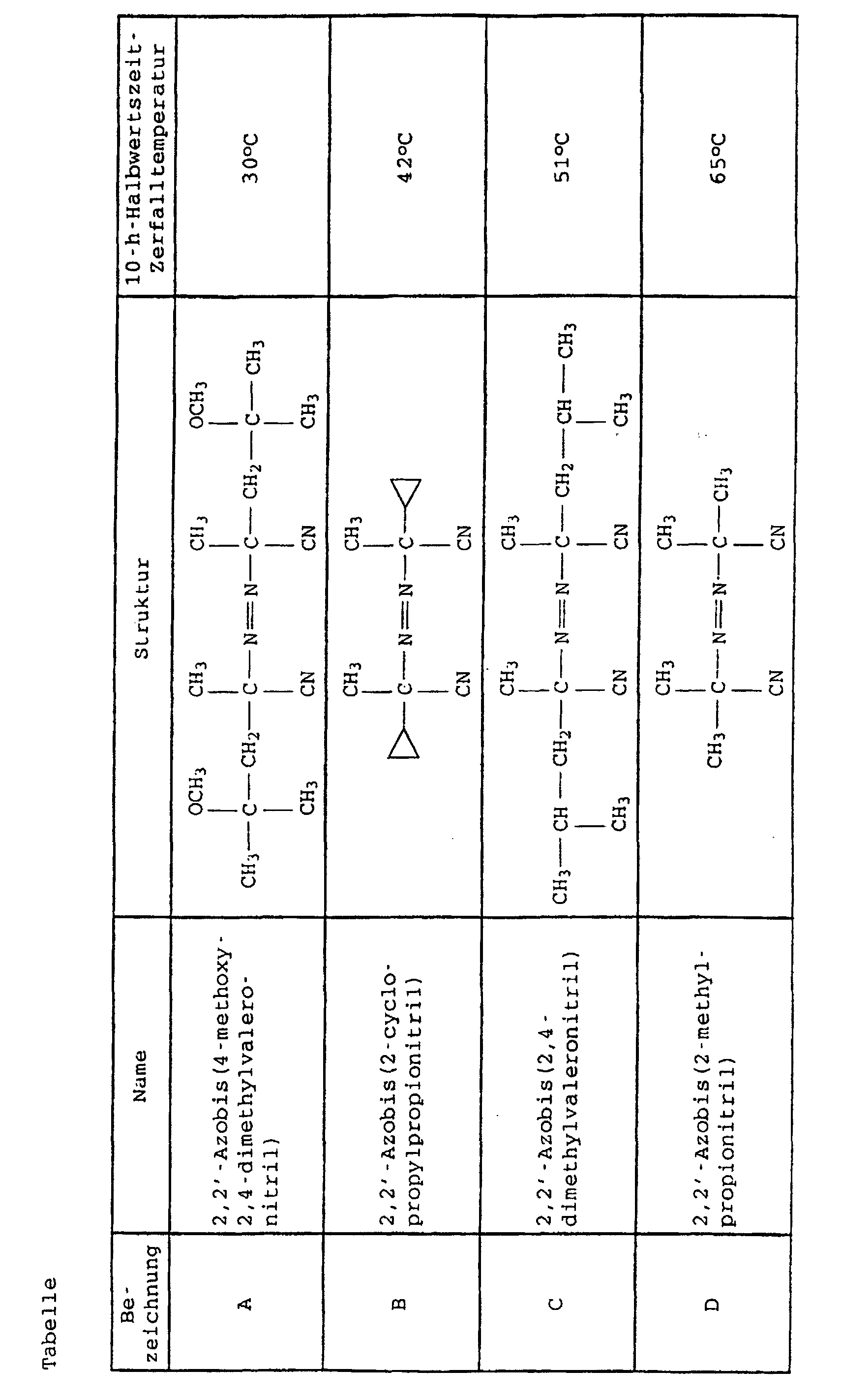

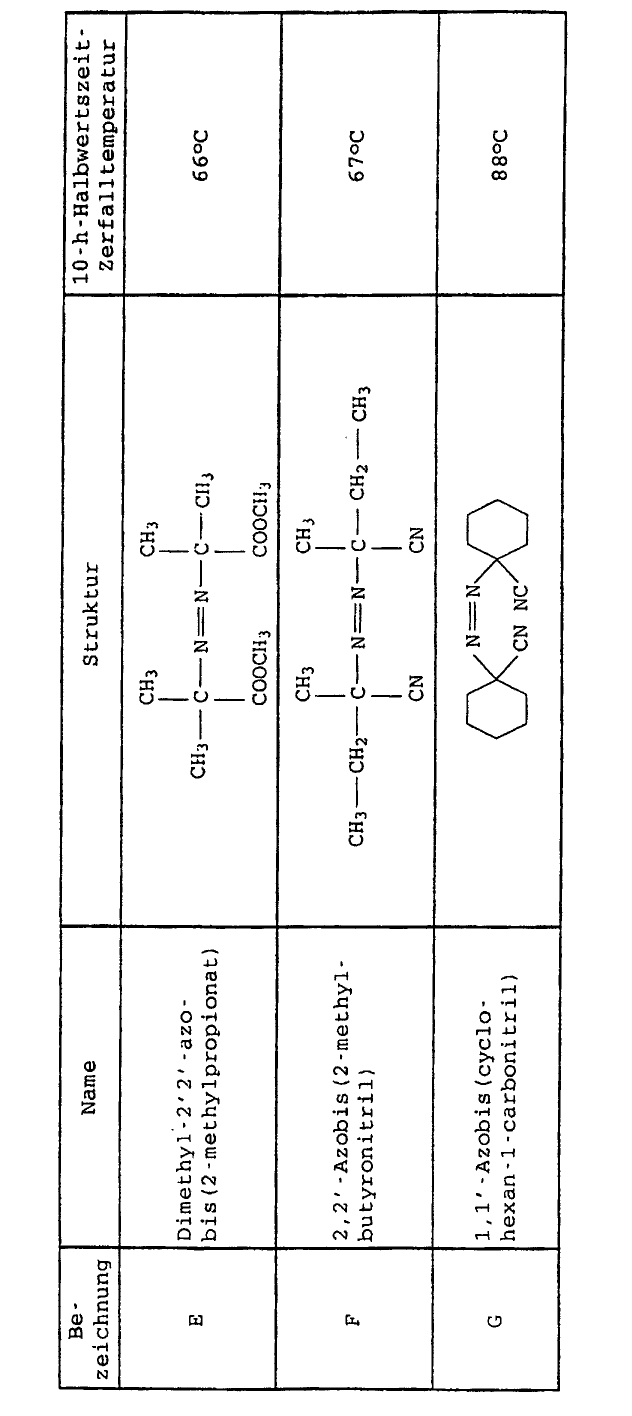

Wie man an Hand der Tabelle erkennen kann, ist es durchaus möglich, empfindliche, leicht zersetzliche Verbindungen nach dem erfindungsgemäßen Verfahren schonend im Temperaturbereich zwischen 20 und 50°C herzustellen, wobei man etwas längere Reaktionszeiten in Kauf nehmen muß, aber das Sicherheitsrisiko einer exothermen Zersetzung ausschalten kann. Es ist überraschend, daß mit organischen Peroxiden als Initiatioren durchweg schlechtere Ergebnisse erhalten werden, während nach dem erfindungsgemäßen Verfahren mit Azoverbindungen in Gegenwart von Oxidationsmitteln sehr gute Resultate erzielt werden. In einigen Fällen kann ein Zusatz einer Mineralsäure, speziell H₂SO₄, von Vorteil sein.

Bevorzugt wird die Bromierung in einem Zweiphasensystem ausgeführt. Im Zweiphasensystem wird im allgemeinen die Lösung des Bromsalzes in Wasser oder bevorzugt die Bromwasserstoffsäure zusammen mit dem verwendeten Lösungsmittel und gegebenenfalls der Initiator bzw. ein Teil der Initiacormenge vorgelegt. Das Reaktionsgemisch wird auf Reaktionstemperatur gebracht und anschließend wird das Toluolderivat II gegebenenfalls in Gegenwart des Initiators kontinuierlich oder portionsweise im Verlauf von einer halben bis mehreren Stunden zudosiert. Parallel zur Dosierung von II erfolgt die Dosierung des Oxidationsmittel im allgemeinen dergestalt, daß in der Reaktionsmischung kein überschüssiges Brom vorhanden ist. Ebenso ist es möglich, das Substrat II zusammen mit dem Bromierungsmittel und dem Initiator vorzulegen und die Reaktion mit der Zudosierung des Oxidationsmittels zu steuern.
Bei der Verwendung von Brom als Bromquelle wird im allgemeinen analog der beschriebenen Verfahrensweise vorgegangen, jedoch Wasser, Lösungsmittel und gegebenenfalls Initiator vorgelegt und Brom zudosiert. Das Substrat II kann bei dieser Fahrweise vorgelegt oder zudosiert werden.
Bei der Verwendung von stabilen Oxidationsmitteln können diese zusammen mit dem Substrat II vorgelegt werden und der Reaktionsablauf durch Zugabe der Bromkomponente gesteuert werden. Beim Einsatz von Wasserstoffperoxid ist die letztgenannte Fahrweise im allgemeinen bis Temperaturen von 50°C möglich.

Die Bromierung läßt sich diskontinuierlich und bevorzugt kontinuierlich durchführen. Die kontinuierliche Fahrweise bietet den Vorteil der geringeren Dimensionierung der verwendeten Apparate und der damit verbundenen geringeren Vorratshaltung der das Substrat II enthaltenden Lösungen bei erhöhter Temperatur. Aufgrund der angesprochenen thermischen Instabilität mancher Toluolderivate II bietet das kontinuierliche Verfahren damit einen sicherheitstechnischen Vorteil.

Nach Ende der Zudosierung wird das Reaktionsgemisch gewöhnlich noch 0,5 bis 3 Stunden bei der gewählten Reaktionstemperatur gehalten. Thermisch stabile Benzylbromide werden in der Regel destillativ aufgereinigt, während thermisch instabile Benzylbromide in der nach dem erfindungsgemäßen Verfahren erhaltenen Lösung weiterverarbeitet werden.

Im Folgenden wird das erfindungsgemäße Verfahren anhand von Beispielen vorgestellt:

### Beispiel 1

Herstellung von o-Nitrobenzylbromid
a) In einem 2,5 Liter Planschliffkolben mit Impellerrührer (300 Upm) und Stromstörer wurde eine Lösung von 6,6 g (1 mol.-% bezogen auf die eingesetzte Bromwasserstoffsäure) Azoisobutyronitril (AIBN) in 1350 g Chlorbenzol zusammen mit 620 g (3,6 mol) 47%iger Bromwasserstoffsäure vorgelegt. Man erwärmte den Reaktorinhalt auf 75°C. Nach Erreichen dieser Temperatur wurden die Zuläufe I und II mittels zweier Dosierpumpen zugefördert.
   Zulauf I: eine Lösung von 26,2 g (4 mol.-%) AIBN in 548 g (4,0 mol) ortho-Nitrotoluol wurde kontinuierlich in 2 Stunden zugefahren;
   Zulauf II: 725 g (3,2 mol) 15%ige H₂O₂ wurde so zugefahren, daß kein überschüssiges Brom in der Lösung vorhanden war.
   Man benötigte hierfür ca. 2,5 Stunden.
   Nach beendetem Zulauf ließ man 2 Stunden bei 75°C nachrühren, stellte dann den Rührer ab und trennte die Phasen bei 75°C. Man erhielt 2146,4 g organischer Phase mit folgender Zusammensetzung (Lösungsmittel nicht berücksichtigt):
   - 60,4 %: o-Nitrobenzylbromid
   - 21,5 %: o-Nitrotoluol
   - 18,2 %: o-Nitrobenzalbromid

   Ausbeute an o-Nitrobenzylbromid: 58,1 % bezogen auf eingesetztes o-Nitrotoluol.
b) In einer 250 ml Rührapparatur wurde eine Mischung aus 25 g Chlorbenzol, in der 13,7 g o-Nitrotoluol und 0,72 g 2,2'-Azobis(4-methoxy-2,4-dimethylvaleronitril) gelöst waren, 0,24 g conc. H₂SO₄ und 7,8 g 50 %-ige H₂O₂ bei 27°C vorgelegt. unter intensiver Rührung wurden in einem Temperaturbereich zwischen 25 und 27°C in einem Zeitraum von ca. 8 Stunden 19,7 g 47 %-ige Bromwasserstoffsäure zugetropft, wobei bei starker Bromfreisetzung (Braunfärbung!) die Zufuhr von Bromwasserstoffsäure unterbrochen wurde und bis zur Entfärbung bei Reaktionstemperatur nachgerührt wurde.
   GC-Analyse der org. Phase:
   - 72,7 %: o-Nitrotoluol
   - 23,9 %: o-Nitrobenzylbromid
   - 0,3 %: o-Nitrobenzalbromid
c) Bei 45°C wurde eine Mischung aus 13,7 g (0,1 mol) o-Nitrotoluol, 25 g Chlorbenzol, 600 mg (2,7 mmol) V 65 (Fa. Wako; 2,2'-Azobis (2,4-dimethyl-valeronitril)), 300 mg H₂SO₄ und 16,4 g (0,15 mol) 30%ige H₂O₂ vorgelegt. Es wurden 10 g 47%ige Bromwasserstoffsäure in 75 min zugetropft und weitere 75 min bei 45°C nachgerührt. Man gab weitere 5 g Bromwasserstoffsäure zu und rührte 12 h bei Raumtemperatur. Dann gab man 3 g Bromwasserstoffsäure und in zwei Portionen 5 g und dann noch einmal 10 g einer Lösung aus Chlorbenzol und V 65 (insgesamt 15 g Chlorbenzol + 0,86 g (3,9 mmol) V 65) zu.
   Qualitatives HPLC der org. Phase:
   - 56,9 %: o-Nitrobenzylbromid
   - 38,4 %: o-Nitrotoluol
   - 4,7 %: o-Nitrobenzalbromid
d) In einer 250 ml-Rührapparatur wurde bei 46 - 48°C eine Mischung aus 13,7 g o-Nitrotoluol, 25 g Chlorbenzol, 0,58 g 2,2'-Azobis(2,4-dimethyl-valeronitril), 0,24 g conc. H₂SO₄ und 19 g 47 %-iger Bromwasserstoffsäure vorgelegt und unter intensivem Rühren in einem Zeitraum von 13 Stunden 60 g 10 %-ige wässrige Wasserstoffperoxid-Lösung zugetropft. Währenddessen wurde portionsweise im stündlichen Abstand von einer Lösung bestehend aus 0,58 g 2,2'-Azobis(2,4-dimethylvaleronitril in 10 g Chlorbenzol jeweils 1 ml zu der Reaktionsmischung zugegeben. Die Reaktion wurde nach 13 Stunden abgebrochen. GC-Analyse der org. Phase:
   - 43,9 %: o-Nitrotoluol
   - 44,2 %: o-Nitrobenzylbromid
   - 1,3 %: o-Nitrobenzalbromid
e) In einer 250 ml Rührapparatur wurde bei 45°C eine Mischung aus 13,7 g o-Nitrotoluol, 35 g Chlorbenzol, 0,24 g conc. H₂SO₄, 1,74 g 2,2'-Azobis (2,4-dimethylvaleronitril) und 8,8 g 30 %-ige wässrige Wasserstoffperoxid-Lösung vorgelegt und unter intensivem Rühren in einem Zeitraum von 24 Stunden 6,6 g Brom in dem Maße zugetropft, wie Entfärbung der braunen Lösung stattfand. GC-Analyse der org. Phase:
   - 52,6 %: o-Nitrotoluol
   - 38,4 %: o-Nitrobenzylbromid
   - 0,9 %: o-Nitrobenzalbromid
f) In einer 250 ml Rührapparatur wurde bei 62°C eine Mischung aus 13,7 g o-Nitrotoluol, 38,8 g Chlorbenzol, 0,24 g conc. H₂SO₄ und 19,8 g 47 %-iger Bromwasserstoffsäure vorgelegt, 2,15 g 2,2' Azobis(2-methylpropionitril) zugegeben und unter intensivem Rühren in einem Zeitraum von 25 Stunden 40 g Chorbleichlauge (12,5 %-ig an aktivem Chlor) langsam zugetropft.
   GC-Analyse der org. Phase
   - 37,4 %: o-Nitrotoluol
   - 44,4 %: o-Nitrobenzylbromid
   - 2,3 %: o-Nitrobenzalbromid
g) In einer 250 ml Rührapparatur wurde eine Mischung aus 13,7 g o-Nitrotoluol, 38,8 g Chlorbenzol, 0,24 g conc. H₂SO₄, 19,8 g 47 %-iger wässriger Bromwasserstoffsäure und 0,72 g 2,2' Azobis(2-methylpropiontril) bei 61°C vorgelegt. Innerhalb von 15 Stunden wurde bei dieser Temperatur eine Lösung von 3,27 g Nacriumbromat (NaBrO₃) in 23 ml Wasser zugetropft. GC-Analyse der org. Phase:
   - 45,7 %: o-Nitrotoluol
   - 44,8 %: o-Nitrobenzylbromid
   - 1,6 %: o-Nitrobenzalbromid
h) Es wurde analog Beispiel g gearbeitet, aber bei einer Reaktionstemperatur von 62 - 63°C und anstatt NaBrO₃ wurde als Oxidationsmittel eine Lösung von 17,5 g Kaliumperoxodisulfat in 50 ml Wasser zu der Reaktionsmischung hinzugegeben. Nach 20 h wurde aufgearbeitet. GC-Analyse der org. Phase:
   - 36,5 %: o-Nitrotoluol
   - 51,6 %: o-Nitrobenzylbromid
   - 2,7 %: o-Nitrobenzalbromid
i) Bei 65°C wurden eine Lösung von 137 g o-Nitrotoluol in 800 ml Chlorbenzol und eine Lösung von 103 g Natriumbromid, 6 g Na₂HPO₄ in 1 l Wasser zusammen mit 2,5 g conc. H₂SO₄ und 20 g 2,2'-Azobis(2-methylpropionitril) vorgelegt. Unter intensiver Rührung dieser zweiphasigen Mischung wurden innerhalb von 2 Stunden 64 g Chlorgas (Cl₂) eingeleitet. GC-Analyse der org. Phase:
   - 34 %: o-Nitrotoluol
   - 50 %: o-Nitrobenzylbromid
   - 4 %: o-Nitrobenzalbromid
j) In einer 250 ml-Rührapparatur wurde bei 62°C eine Mischung aus 13,7 g o-Nitrotoluol, 38,8 g Chlorbenzol, 0,24 g conc. H₂SO₄ und 19,8 g 47 %-iger wässriger Bromwasserscoffsäure vorgelegt, 2,32 g 2,2'-Azobis(2-methylpropionitril) zugegeben und unter intensivem Rühren in einem Zeitraum von 25,5 Stunden 11,6 g Peroxyessigsäure (32 %ig) in kleinen Portionen zugetropft.
   GC-Analyse der org. Phase:
   - 53,5 %: o-Nitrotoluol
   - 32,2 %: o-Nitrobenzylbromid
   - 0,5 %: o-Nitrobenzalbromid

### Beispiel 2: Herstellung von 3-Chlor-2-Brombenzylbromid

In einer 2 1-Rührapparatur wurde eine Mischung aus 267,7 g 2-Brom-3-chlortoluol, 530 g Chlorbenzol, 2,5 g conc. H₂SO₄ 257,9 g 47 %-iger Bromwasserstoffsäure und 14 g 2,2'Azobis(2-methylpropionitril) bei 63°C vorgelegt. Innerhalb von einer Stunde und 25 Minuten wurden 332,3 g einer 10 %-igen wässrigen Wasserstoffperoxid-Lösung zugetropft, dann wurde 30 Minuten bei 63°C nachgerührt. GC-Analyse der org. Phase:
- 32,2 %: 3-Chlor-2-bromtoluol
- 56,5 %: 3-Chlor-2-brombenzylbromid
- 4,2 %: 3-Chlor-2-brombenzalbromid

Diese 3 Komponenten ließen sich über eine Destillation problemlos trennen und auf reinigen.

### Beispiel 3: Herstellung von 3-Methyl-2-brombenzylbromid

In einer 1 l-Rührapparatur wurde 104,5 g 2-Brom-m-xylol(2,6-Dimethylbrombenzol), 200 g Chlorbenzol, 1 g conc. H₂SO₄, 87,2 g 47 %-ige Bromwasserstoffsäure und 6 g 2,2'-Azobis(2-methylpropionitril) bei 63°C vorgelegt. Innerhalb von 30 Minuten wurden 75 g 10 %-ige wässrige Wasserstoffperoxid-Lösung zugetropft und 25 Minuten bei 64°C nachgerührt.
GC-Analyse der org. Phase:
- 40,7 %: 2,6-Dimethylbrombenzol
- 45,8 %: 3-Methyl-2-brombenzylbromid
- 0,6 %: 3-Methyl-2-brombenzalbromid
- 3,0 %: 2,6-Bis-(brommethyl)brombenzol

Auch hier konnte die Benzylbromidkomponente über eine Destillation abgetrennt und in Reinform gewonnen werden.

### Beispiel 4: Herstellung von 4-Chlor-2-fluorbenzylbromid

In einer 4 l-Rührapparatur wurden 361,5 g 4-Chlor-2-fluortoluol, 520 g Chlorbenzol, 6 g conc. H₂SO₄, 467 g 47 %-ige Bromwasserstoffsäure und 2,7 g 2,2'Azobis(2-methylpropionitril) bei 70°C vorgelegt. Innerhalb von 1,5 Stunden wurden gleichzeitig 620,5 g einer 10 %-igen wässrigen Wasserstoffperoxid-Lösung und eine Lösung von 16,5 g 2,2'Azobis(2-methylpropionitril) in 270 g Chlorbenzol zugetropft. Danach wurde 1 Stunde bei 70°C nachgerührt.
GC-Analyse der ogr. Phase:
- 31,2 %: 4-Chlor-2-fluortoluol
- 60,5 %: 4-Chlor-2-fluorbenzylbromid
- 4,3 %: 4-Chlor-2-fluorbenzalbromid

Die Reinigung konnte auch in diesem Falle mit Hilfe einer fraktionierten Destillation vorgenommen werden.

### Beispiel 5: Herstellung von 2,4-Dichlor-3-(brommethyl)benzoesäuremethylester

In einer 1 l Rührapparatur wurden 94,6 g 2,4-Dichlor-3-methylbenzoesäuremethylester, 315 g Chlorbenzol, 1 g conc. H₂SO₄, 85,5 g 47 %-ige Bromwasserstoffsäure und 3,5 g 2,2'Azobis(2-methylpropionitril) bei 63°C vorgelegt. Nun wurden bei Reaktionstemperaturen zwischen 63 und 68°C innerhalb von 35 Minuten 73,5 g 10 %-ige wässrige Wasserstoffperoxid-Lösung zugegeben. Es wurde 2 Stunden bei Reaktionstemperatur nachgerührt, dann wurden nochmals 73,5 g 10 %-ige Wasserstoffperoxid-Lösung in 30 Minuten zugetropft, weitere 1,5 g 2,2'-Azobis(2-methylpropionitril) der Reaktion zugeführt, 2 Stunden und 35 Minuten bei Reaktionstemperatur nachgerührt und zum Schluß 36,8 g 10 %-ige Wasserstoffperoxid-Lösung in 15 Minuten bei 63 - 67°C zugetropft, wiederum 2 Stunden bei Reaktionstemperatur nachgerührt, auf Raumtemperatur abgekühlt und die organische Phase abgetrennt. In der Chlorbenzol-Lösung lag das Wertprodukt 2,4-Dichlor-3-brommethyl-benzoesäuremethylester in einer Reinheit von 96,1 % (nach HPLC-Analyse, Lösungsmittel wurde nicht berücksichtigt) vor.

## Patentansprüche

1. Verfahren zur Herstellung von substituierten Benzylbromiden der allgemeinen Formel I 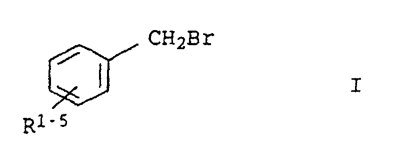 in der mindestens ein Substituent R¹⁻⁵ eine elektronenziehende Gruppe wie Fluor, Chlor, Brom, C₁-C₄-Alkoxycarbonyl, Cyano oder Nitro bedeutet und die übrigen Substituenten R¹⁻⁵ Wasserstoff oder Methyl bedeuten,
durch Bromierung von substituierten Toluolen der allgemeinen Formel II 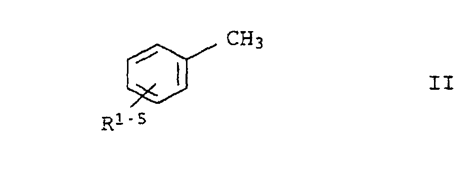 mit einem Bromierungsmittel ausgewählt aus der Gruppe: Brom, Bromsalze oder Bromwasserstoff, gegebenenfalls in Form seiner wäßrigen Lösung, bei Temperaturen von 20 bis 95°C, **dadurch gekennzeichnet, daß** die Bromierung in Gegenwart eines Azocarbonsäurenitrils oder eines Azocarbonsäureesters und in Gegenwart eines Oxidationsmittels durchgeführt wird.

2. Verfahren gemäß Anspruch 1, **dadurch gekennzeichnet, daß** im Falle von elementarem Brom als Bromierungsmittel 0.5 bis 1 Äquivalent eines Oxidationsmittels und im Falle von Bromiden oder Bromwasserstoff 1,5 bis 2 Äquivalente eines Oxidationsmittel eingesetzt werden.

3. Verfahren gemäß Anspruch 1, **dadurch gekennzeichnet, daß** als Oxidabionmittel Wasserstoffperoxid, Peressigsäure, Chlor, Natriumhypochlorit (Chlorbleichlauge), Natriumbromat oder Kaliumperoxydisulfat verwendet werden.

4. Verfahren gemäß Anspruch 1, **dadurch gekennzeichnet, daß** als Oxidationsmittel Wasserstoffperoxid verwendet wird und dieses bei Temperaturen bis 50°C zusammen mit dem zu bromierenden Substrat vorgelegt wird.

5. Verfahren gemäß den Ansprüchen 1 bis 4, **dadurch gekennzeichnet, daß** die Bromierung in einem Zweiphasensystem durchgeführt wird.

6. Verfahren gemäß den Ansprüchen 1 bis 5, **dadurch gekennzeichnet, daß** die Bromierung kontinuierlich durchgeführt wird.

## Claims

1. A process for preparing substituted benzyl bromides of the formula I 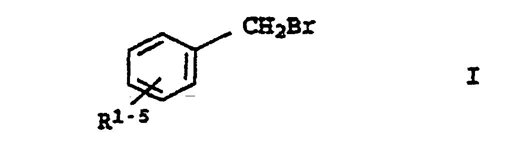 where at least one substituent R¹⁻⁵ is an electron-attracting group such as fluorine, chlorine, bromine, C₁-C₄-alkoxycarbonyl, cyano or nitro, and the other substituents R¹⁻⁵ are hydrogen or methyl, by bromination of substituted toluenes of the formula II 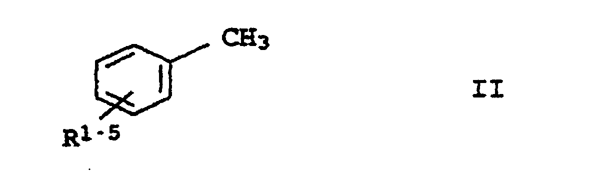 with a brominating agent selected from the group: bromine, bromine salts or hydrogen bromide where appropriate in the form of its aqueous solution, at from 20 to 95°C, wherein the bromination is carried out in the presence of an azo carbonitrile or of an azo carboxylic ester and in the presence of an oxidizing agent.

2. A process as claimed in claim 1, wherein from 0.5 to 1 equivalent of an oxidizing agent is employed in the case of elemental bromine as brominating agent, and from 1.5 to 2 equivalents of an oxidizing agent are employed in the case of bromides or hydrogen bromide.

3. A process as claimed in claim 1, wherein hydrogen peroxide, peracetic acid, chlorine, sodium hypochlorite (chlorine bleaching solution), sodium bromate or potassium peroxydisulfate are used as oxidizing agent.

4. A process as claimed in claim 1, wherein hydrogen peroxide is used as oxidizing agent and is initially present together with the substrate to be brominated at up to 50°C.

5. A process as claimed in any of claims 1 to 4, wherein the bromination is carried out in a two-phase system.

6. A process as claimed in any of claims 1 to 5, wherein the bromination is carried out continuously.

## Revendications

1. Procédé pour la préparation de bromures de benzyle substitués de formule générale I 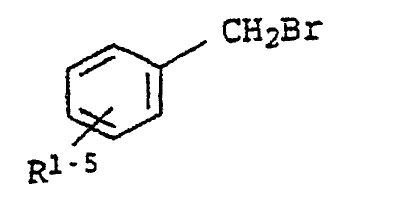 dans laquelle au moins un substituant R¹⁻⁵ représente un groupe attirant les électrons tel qu'un atome de fluor, chlore, brome ou un groupe alcoxy(C1-C4)carbonyle, cyano ou nitro, et les autres substituants R¹⁻⁵ représentent un atome d'hydrogène ou le groupe méthyle, par bromation de toluènes substitués de formule générale II 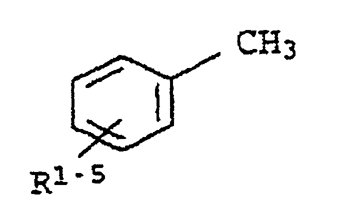 avec un agent de bromation choisi dans le groupe constitué par : le brome, des sels de brome et le bromure d'hydrogène, éventuellement sous forme de sa solution aqueuse, à des températures de 20 à 95°C,
**caractérisé en ce que** la bromation est effectuée en présence d'un azocarbonitrile ou d'un ester d'acide azocarboxylique et en présence d'un oxydant.

2. Procédé selon la revendication 1, **caractérisé en ce que** dans le cas du brome élémentaire en tant qu'agent de bromation, on utilise de 0,5 à 1 équivalent d'un oxydant, et dans le cas de bromures ou d'acide bromhydrique, on utilise de 1,5 à 2 équivalents d'un oxydant.

3. Procédé selon la revendication 1, **caractérisé en ce qu'**on utilise comme oxydant le peroxyde d'hydrogène, l'acide peracétique, le chlore, l'hypochlorite de sodium (solution de chlore décolorant), le bromate de sodium ou le peroxodisulfate de potassium.

4. Procédé selon la revendication 1, **caractérisé en ce qu'**on utilise comme oxydant le peroxyde d'hydrogène et on le dispose au préalable à des températures jusqu'à 50°C, conjointement avec le substrat bromé.

5. Procédé selon les revendications 1 à 4, **caractérisé en ce que** la bromation est effectuée dans un système en deux phases.

6. Procédé selon les revendications 1 à 5, **caractérisé en ce que** la bromation est effectuée en continu.
